# EUROPEAN PATENT SPECIFICATION

(11) **EP 4 240 300 B1**
(45) Date of publication and mention of the grant of the patent: **18.03.2026**
(21) Application number: 21810732.4
(22) Date of filing: 29.10.2021
(51) Int. Cl.: A61F 9/007

(54) **CONTROLLING INTRAOCULAR PRESSURE DURING PHACOEMULSIFICATION PROCEDURES**
KONTROLLE DES INTRAOKULAREN DRUCKS WÄHREND PHAKOEMULSIFIKATIONSVERFAHREN
CONTRÔLE DE LA PRESSION INTRAOCULAIRE PENDANT DES PROCÉDURES DE PHACOÉMULSIFICATION

(30) Priority: 05.11.2020 US 202063110186 P; 25.10.2021 US 202117510052
(43) Date of publication of application: 13.09.2023
(73) Proprietor: Johnson & Johnson Surgical Vision, Inc., Irvine, CA 92618 (US)
(72) Inventor: GOVARI, Assaf, 2066717 Yokneam (IL); ALGAWI, Yehuda, 2066717 Yokneam (IL); SITNITSKY, Ilya, 2066717 Yokneam (IL); KATZIR, Stanislav, 2066717 Yokneam (IL)
(74) Representative: Carpmaels & Ransford LLP
(86) International application number: PCT/IB2021/060052
(87) International publication number: WO 2022/097004

(56) References cited:
- US-A- 4 832 685
- US-A- 5 328 456
- US-A1- 2013 150 782
- US-A1- 2014 114 237
- US-A1- 2014 163 455
- US-A1- 2014 257 172
- US-A1- 2017 367 885
- US-A1- 2020 107 958

## Description

### FIELD OF THE INVENTION

The present invention relates generally to medical procedures, and particularly to methods and systems for controlling parameters in phacoemulsification procedures.

### BACKGROUND OF THE INVENTION

A cataract is a clouding and hardening of the eye's natural lens, a structure which is positioned behind the cornea, iris and pupil. The lens is mostly made up of water and protein and as people age these proteins change and may begin to clump together obscuring portions of the lens. To correct this, a physician may recommend phacoemulsification cataract surgery. In the procedure, a physician makes a small incision in the sclera or cornea of the eye. Then a portion of the anterior surface of the lens capsule is removed to gain access to the cataract. The physician then uses a phacoemulsification probe, which has an ultrasonic handpiece with a needle. The tip of the needle vibrates at ultrasonic frequency to sculpt and emulsify the cataract while a pump aspirates particles and fluid from the eye through the tip. Aspirated fluids are replaced with irrigation of a balanced salt solution (BSS) to maintain the anterior chamber of the eye, and to maintain constant intraocular pressure (IOP) and temperature in the eye during the procedure. After removing the cataract with phacoemulsification, the softer outer lens cortex is removed with suction. An intraocular lens (IOL) is then introduced into the empty lens capsule restoring the patient's vision.

Various techniques of irrigation and aspiration using a phacoemulsification probe have been proposed in the patent literature. For example, U.S. Patent Application Publication no. 2014/0163455 describes a method of irrigating a surgical site and aspirating fluid from the surgical site. The method includes directing a fluid through an aspiration conduit in a phacoemulsification hand piece using a vacuum pressure created from a pump in the hand piece interfacing with the aspiration conduit and directing an irrigation fluid through an irrigation conduit in the hand piece using a pressure created from the pump interfacing with the irrigation conduit. The method also includes increasing an irrigation fluid flow through the irrigation conduit by activating the pump in the hand piece, detecting a pressure associated with a surgical site using a sensor, and controlling intraocular pressure (IOP) by adjusting the pump speed based on the detected pressure.

U.S. Patent Application Publication no. 2009/0163863 describes a surgical system for aspiration of a biological material comprising a source of irrigation fluid, a collection cassette, a pump for creating a vacuum in the collection cassette, a handpiece applied to a surgical area for infusing irrigation fluid and for aspirating a biological material, conduits, and connecting the handpiece to each of the source of irrigation fluid and the collection cassette, and means for stopping the pump and preventing creation of vacuum within the conduit and collection cassette after receiving a stop signal.

U.S. Patent Application Publication no. 2014/0114237 describes a surgical system comprises a pressurized irrigation fluid source, an irrigation line fluidly coupled with the pressurized irrigation fluid source, a handpiece fluidly coupled with the irrigation line, an irrigation pressure sensor located at or along the pressurized irrigation fluid source or irrigation line, and a controller for controlling the pressurized irrigation fluid source. The controller controls the pressurized irrigation fluid source based on a reading from the irrigation pressure sensor and an estimated flow value modified by a compensation factor.

U.S. Patent no. 10,278,861 describes a phacoemulsification system that includes a hand-graspable body and a phacoemulsification needle extending from a distal portion of the body. The phacoemulsification system may also include an impeller pump carried by the hand-graspable body and configured to convey an aspiration fluid from a surgical site. The impeller pump may include an aspiration motor and a flexible impeller coupled with and rotatably driven by the aspiration motor. The flexible impeller may be arranged to aspirate fluid and emulsified lens tissue from the surgical site.

U.S. Patent no. 9,549,851 describes a surgical handpiece comprising a shell, a channel having an irrigation conduit, and a sensor housing. The channel is coupled with the shell such that the proximal end of the channel is located at the proximal end of the shell and the distal end of the channel is located near the distal end of the shell. The sensor housing has an irrigation path extending through it. The sensor housing has a seal interface on one end. The seal interface end of the sensor housing is coupled with the proximal end of the channel such that the irrigation conduit of the channel is fluidly coupled with the irrigation path of the sensor housing.

Chinese Patent Application no. 11,033,897 described an ultrasonic emulsification handle with a sensor and a surge control system and method.

U.S. Patent Application Publication no. 2020/0107958 describes a system for learning actual phacoemulsification energy setpoints at the time of occlusion breaks during ophthalmic surgery. Phacoemulsification energy setpoints may be recorded over different periods of time, such as during current cases and over a lifetime of cases. The recorded data would then be used to determine future phacoemulsification energy setting amounts.

U.S. Patent Application Publication no. 2019/0099529 describes variables of a surgical system that are detected or received via one or more sensors to predict an intraocular pressure (IOP) and/or determine an IOP in real time during a surgical procedure. A notification to a surgeon or a target IOP is set and maintained as determined by static IOP, dynamic IOP, and/or a total IOP combining both static and dynamic IOP of the anterior chamber of a patient's eye. Information collected about various components of the system are displayed on a user interface. The system uses the collected information to calculate the static IOP and/or dynamic IOP of the system, and the total IOP may be a function of the static IOP and/or dynamic IOP measurements.

U.S. Patent no. 10,500,319 describes a surgical handpiece having a source of ultrasonic energy provided to a connecting body having a first passage with one end to receive fluid from a first source and the other end at the connecting body distal end. A work tip has first and second tubes each having at least one opening at its distal end and the proximal end of one or both of the tubes connected to the connecting body distal end to receive the ultrasonic energy and to selectively receive or discharge fluid from either the first or second source as controlled by a valve. A cannula is provided which is adapted to be placed in an opening in the cornea through which the work tip extends during the removal of cataracts from the eye by phacoemulsification. The cannula may be equipped with channels that permit additional infusion of fluid into the eye during the procedure, which infusion also cools the surrounding corneal tissue to protect against heat generated by the ultrasonic vibration of the work tip.

U.S. Patent Application Publication no. 2019/0143008 describes a system and method for a diaphragm-based fluidics aspiration system. The aspiration system includes a waste reservoir configured to hold a vacuum pressure, an aspiration connection, a vacuum pinch valve including a first outlet fluidically coupled with the waste reservoir, a second outlet fluidically coupled with the aspiration connection; a fluid channel, and an actuator configured to alter a cross-sectional area of the fluid channel.

U.S. Patent no. 9,931,447 describes apparatuses, systems, and methods for rapid venting of vacuum to mitigate occlusion break surge within a phacoemulsification fluidics aspiration system. The aspiration system may utilize a positive displacement pump to generate flow and a vent valve to vent or moderate the pressure or vacuum level based on pressure sensor feedback detected within the eye and at the pump. Embodiments described herein provide a quick-opening vent valve that produces a quick-opening flow response upon angular rotation of the quick-opening vent valve. The flow performance at the quick-opening vent valve is improved by increasing the effective cross-sectional area of the valve at near-closed valve positions.

U.S. Patent Application Publication no. 2017/0312431 describes an ophthalmic surgical system enabling pressure measurements proximal to the eye may include an illumination probe having a probe tip configured for insertion through an incision in an eye of a patient. The probe tip may include a distal end of an optical fiber, wherein the distal end of the optical fiber includes a first reflective surface and a second reflective surface. The first and second reflective surfaces being separated by a cavity, wherein the second reflective surface is provided by a partially transparent wall forming an exterior surface of the distal end of the optical fiber.

U.S. Patent Application Publication no. 2018/0279876 describes a system for measuring intraocular pressure in an eye of a patient, which includes a sensor configured to be positioned in the eye of the patient. The sensor includes a sealed cavity, and a flexible membrane sealing a distal end of the sealed cavity, the flexible membrane configured to deflect responsive to the intraocular pressure in the eye of the patient. The system includes a detection device configured to be positioned external to the eye of the patient and optically coupled with the sensor, the detection device configured to detect an indication of change in length of the sealed cavity resulting from deflection of the flexible membrane.

U.S. Patent Application Publication no. 2014/0257172 describes a fluidics system including an irrigation system and an aspiration system in fluid communication with a phacoemulsification handpiece. The irrigation system may also include an irrigation fluid pressure sensor which is operable to sense a pressure of the irrigation fluid.

### SUMMARY OF THE INVENTION

The scope of the present invention is defined by the set of claims that follows. Any examples described, which do not fall under the scope of the claims, are given for illustrative purposes only. An embodiment of the present invention that is described herein provides a phacoemulsification system, including a phacoemulsification probe and a sensing assembly. The phacoemulsification probe includes: (i) a needle, which is configured to be inserted into a lens capsule of an eye and to be vibrated to emulsify a lens of the eye, (ii) an irrigation channel, configured for flowing irrigation fluid into the lens capsule, and (iii) an aspiration channel, configured for removing at least eye fluid from the lens capsule. The sensing assembly is coupled with a proximal end of the phacoemulsification probe and is configured to sense a pressure of at least one of the irrigation fluid and the eye fluid.

The sensing assembly includes a pressure sensing assembly (PSA) coupled between an aspiration tube and the aspiration channel and configured to sense the pressure of the eye fluid flowing between the aspiration channel and the aspiration tube. The PSA includes: (i) a hollow tube, which is inserted between the aspiration channel and the aspiration tube, and is configured to flow at least the eye fluid, and (ii) a pressure sensing device (PSD), which is placed in direct contact with the eye fluid flowing in the hollow tube and is configured to produce a pressure signal indicative of the pressure of the eye fluid flowing between the aspiration channel and the aspiration tube. The hollow tube includes, a first luer connector coupled with the aspiration channel, and a second luer connector coupled with the aspiration tube, and , the PSD is positioned between the first luer connector and the second luer connector.

In an embodiment, the phacoemulsification system further including a wire, which is connected to the PSD and is configured to transmit the pressure signal to a processor of a phacoemulsification console. In another embodiment, the sensing assembly includes a temperature sensor coupled with the aspiration channel and configured to sense a temperature of the eye fluid at the proximal end of the phacoemulsification probe.

There is additionally provided, as example, a phacoemulsification system, including a phacoemulsification probe, a rotatable valve, one or more sensors and a processor. The phacoemulsification probe, includes: (i) a needle, which is configured to be inserted into a lens capsule of an eye and to be vibrated to emulsify a lens of the eye, (ii) an irrigation channel for flowing irrigation fluid into the lens capsule, and (iii) an aspiration channel for removing at least eye fluid from the lens capsule. The rotatable valve includes solenoids wrapped around a tube including a shaft having electric dipole. The rotatable valve is disposed between at least two of the irrigation channel, an irrigation tube, and an aspiration tube, and is configured, in response to applying electrical current to the solenoids, to rotate the shaft and the tube for toggling between at least: (i) a first position that enables flow of at least part of the irrigation fluid into the aspiration tube, and (ii) a second position that blocks the flow of the irrigation fluid into the aspiration tube and enables the flow of the irrigation fluid into the eye through the irrigation channel. The one or more sensors are configured to measure fluid pressure at a distal portion of one or both of the irrigation channel and the aspiration channel. The processor is configured, based on the fluid pressure measured by the one or more sensors, to control a rotation of the rotatable valve for toggling between the first and second positions by altering a current supplied to one or more of the solenoids.

In some examples, at least one of the one or more sensors is disposed within the phacoemulsification probe. In other examples, the phacoemulsification system further includes an aspiration bypass, which is coupled between the rotatable valve and the aspiration tube, and is configured to flow the irrigation fluid into the aspiration tube.

There is further provided, in accordance with an example, a method including: in a phacoemulsification system that includes: (i) a phacoemulsification probe, including: (a) a needle, which is configured to be inserted into a lens capsule of an eye and to be vibrated to emulsify a lens of the eye, (b) an irrigation channel for flowing irrigation fluid into the lens capsule, and (c) an aspiration channel for removing at least eye fluid from the lens capsule, (ii) a rotatable valve including solenoids wrapped around a tube including a shaft having electric dipole, the rotatable valve is disposed between at least two of the irrigation channel, an irrigation tube and an aspiration tube, and is configured, in response to applying electrical current to the solenoids, to rotate the shaft and the tube for toggling between at least: (a) a first position that enables flow of at least part of the irrigation fluid into the aspiration tube, and (b) a second position that blocks the flow of the irrigation fluid into the aspiration tube and enables the flow of the irrigation fluid into the eye through the irrigation channel, receiving measurement of fluid pressure at a distal portion of one or both of the irrigation channel and the aspiration channel. Based on the fluid pressure measurement, a rotation of the rotatable valve is controlled for toggling between the first and second positions by altering a current supplied to one or more of the solenoids.

There is additionally provided, in accordance with an embodiment of the present invention, a phacoemulsification system, including a phacoemulsification probe, and a connector assembly. The phacoemulsification probe includes: (i) a needle, which is configured to be inserted into a lens capsule of an eye and to be vibrated to emulsify a lens of the eye, (ii) an irrigation channel for flowing irrigation fluid into the lens capsule, and (iii) an aspiration channel for removing at least eye fluid from the lens capsule. The connector assembly, includes: (a) a housing, including: (i) an irrigation passage, connecting between the irrigation channel and an irrigation tube and configured to pass the irrigation fluid into the irrigation channel, and (ii) an aspiration passage, connecting between the aspiration channel and an aspiration tube and configured to pass at least the eye fluid away from the aspiration channel to the aspiration tube, (b) one or more sensors, contained in the housing and configured to measure at least one of fluid pressure and fluid temperature at one or both of the irrigation channel and the aspiration channel, and (c) an electrical connector, which is configured to connect and disconnect the connector assembly and the phacoemulsification probe, and when the connector assembly is disconnected from the phacoemulsification probe, operation of the phacoemulsification probe is disabled.

In some embodiments, the one or more sensors include a first pressure sensor disposed in the irrigation passage and a second pressure sensor disposed in the aspiration passage.

There is further provided, in accordance with an embodiment of the present invention, a method for producing a connector assembly, the method includes, receiving a housing, including: (i) an irrigation passage, connecting between an irrigation channel of a phacoemulsification probe and an irrigation tube for passing irrigation fluid into the irrigation channel, and (ii) an aspiration passage, connecting between an aspiration channel and an aspiration tube for passing at least an eye fluid away from the aspiration channel to the aspiration tube. One or more sensors for measuring at least one of fluid pressure and fluid temperature at one or both of the irrigation channel and the aspiration channel, are disposed in the housing. An electrical connector, for connecting and disconnecting the connector assembly and the phacoemulsification probe, is coupled with the housing, and, when the connector assembly is disconnected from the phacoemulsification probe, operation of the phacoemulsification probe is disabled. In some embodiments, disposing the one or more sensors includes disposing: (i) a first pressure sensor in the irrigation passage, and (ii) a second pressure sensor in the aspiration passage.

There is additionally provided, as example, an ophthalmic handpiece, including a distal section and a proximal section. The distal section is configured to be inserted into an eye of a patient and includes a pressure sensor configured to produce a signal indicative of a fluid pressure in the eye. The proximal section is configured to be connected with and disconnected from the distal section, and includes a wireless communication device configured to transmit the signal to an external system.

In some examples, the ophthalmic handpiece further includes a substrate having one or more electrical traces, the wireless communication device is mounted on the substrate, and when the proximal section and the distal section are connected, the one or more electrical traces are configured to conduct the signal from the pressure sensor to the wireless communication device.

In other examples, the ophthalmic handpiece further includes a battery, which is configured to supply power to the wireless communication device. In yet other embodiments, the ophthalmic handpiece further includes a sterile sleeve, which is wrapped around the proximal section, and is configured to seal the proximal section, so as to enable reuse of the proximal section.

The present invention will be more fully understood from the following detailed description of the embodiments thereof, taken together with the drawings in which:

### BRIEF DESCRIPTION OF THE DRAWINGS

Fig. 1 is a schematic, pictorial illustration of a phacoemulsification system, in accordance with an embodiment of the present invention;
Fig. 2 is a schematic, pictorial illustration of a pressure sensing assembly (PSA) coupled with a phacoemulsification probe, in accordance with an embodiment of the present invention;
Fig. 3 is a schematic, pictorial illustration of sensors disposed at the proximal end of a phacoemulsification probe, in accordance with another embodiment of the present invention;
Fig. 4 is a schematic, pictorial illustration of a connector assembly of a phacoemulsification system, in accordance with another embodiment of the present invention;
Fig. 5 is a schematic, pictorial illustration of a rotatable valve of a phacoemulsification probe, in accordance with another embodiment of the present invention; and
Fig. 6 is a schematic, pictorial illustration of an ophthalmic handpiece (OHP) configured for measuring intraocular pressure (IOP) in a patient's eye, in accordance with another embodiment of the present invention.

### DETAILED DESCRIPTION OF EMBODIMENTS

### OVERVIEW

During phacoemulsification of an eye lens, the emulsified lens particles are aspirated. When a particle blocks the inlet of the aspiration channel the vacuum in the line increases. When the line becomes unblocked (e.g., by the particle being subsequently sucked into the line), the high vacuum in the line may cause an aspiration surge with potentially traumatic consequences to the eye.

A possible solution to the problem of vacuum level surge is to incorporate an aspiration bypass. Such a bypass may consist of a small hole or channel between the irrigation channel and the aspiration channel. When a blockage occurs, the high vacuum diverts irrigation fluid into the aspiration channel via the hole, thereby limiting the vacuum level.

However, the above-described bypass aspiration technique is still prone to produce a traumatic aspiration surge when the line unblocks, since the high vacuum is present in a long line between a portion of the aspiration channel inside the emulsification probe and the aspiration pump, and that large, partially vacant volume (or even when the channel is full with fluid), may therefore cause a surge. Moreover, diversion of irrigation fluid may cause an uncontrolled drop of pressure in the irrigation channel, which may also pose a risk to the eye.

Examples related to the present invention that are described hereinafter provide improved techniques for controlling and maintaining constant intraocular pressure (IOP) in a patient's eye undergoing a phacoemulsification procedure and to reduce and/or eliminate post occlusion surge. In some embodiments, a phacoemulsification system, comprises a phacoemulsification probe and a processor. The phacoemulsification probe comprises a needle, which is configured to be inserted into a lens capsule of the treated eye and to be vibrated to emulsify a lens of the eye. The phacoemulsification probe further comprises an irrigation channel, configured for flowing irrigation fluid into the lens capsule, and an aspiration channel, configured for removing eye fluid and lens material from the capsular bag.

In some examples, the phacoemulsification system comprises a sensing assembly, which is coupled with a handle located at the proximal end of the phacoemulsification probe. Note that sensing in close proximity to the patient's eye, improves the response-time to events of pressure drop or pressure surge during the procedure, so as to maintain constant IOP in the patient's eye, as described above.

In some examples, the sensing assembly is configured to sense various parameters, such as pressure and temperature of one or both of the irrigation fluid and the eye fluid. The sensing assembly may comprise a pressure sensing assembly (PSA) coupled between an aspiration tube and the aspiration channel of the phacoemulsification probe, and configured to sense the pressure of the eye fluid flowing between the aspiration channel and the aspiration tube. The PSA may be designed to be reusable or disposable. Similarly, the sensing assembly may comprise a temperature sensor coupled with the aspiration channel and configured to sense the temperature of the eye fluid at the proximal end of the phacoemulsification probe.

In some examples, the phacoemulsification system may have a rotatable valve, which may be an add-on module, or designed into the phacoemulsification probe. The rotatable valve may comprise solenoids wrapped around a tube having one or more shafts, which are made from magnetic material such as ferrite and serve as magnets for toggling described herein. The rotatable valve is disposed between the irrigation channel, the irrigation tube and the aspiration tube, and is configured to rotate for toggling between a first position (applied, for example, when at least one of the aspiration channel and aspiration tube is obstructed by lens material) that enables flow of at least part of the irrigation fluid into the aspiration tube, and a second position (applied, for example, when both aspiration channel and aspiration tube are open (e.g. unobstructed) that blocks the flow of the irrigation fluid into the aspiration tube and allows flow of the irrigation fluid from the irrigation tube to the irrigation channel.

In some examples, the processor is configured, based on the fluid pressure measured by the PSA, to control the rotatable valve to toggle between the first and second positions by altering a current supplied to one or more of the solenoids. Note that using the solenoids enables transition time of the order of a fraction of a millisecond, between the first and second positions, so as to maintain constant IOP during the phacoemulsification procedure.

In other examples, the phacoemulsification system comprises a connector assembly, having a housing, multiple sensors, and an electrical connector. The housing comprising (i) an irrigation passage, connecting between the irrigation channel and the irrigation tube and configured to pass the irrigation fluid into the irrigation channel, and (ii) an aspiration passage, connecting between the aspiration channel and the aspiration tube and configured to pass the eye fluid from the aspiration channel to the aspiration tube.

In some examples, the sensors are contained in the housing and are configured to measure fluid pressure, and optionally the fluid temperature, at one or both of the irrigation channel and the aspiration channel. The electrical connector is configured to connect and disconnect between the housing and the phacoemulsification probe, and, when disconnected from the phacoemulsification probe, the electrical connector is configured to disable the operation of the phacoemulsification probe so as to prevent emulsification of the eye lens without controlled irrigation and aspiration as described above. In other embodiments, the housing may comprise a valve, which is controlled by the processor and is configured to control the flow level of one or both of the irrigation and aspiration in response to pressure and/or temperature signals received from the sensors.

As described above, it is important to maintain constant IOP during a phacoemulsification procedure. In some examples, an ophthalmic handpiece described herein may be coupled with the phacoemulsification probe or used as a stand-alone tool. The ophthalmic handpiece may comprise a distal section, which is typically disposable, and a pluggable proximal section, which is typically reusable. The distal section is configured to be inserted into the eye in question and comprises a pressure sensor configured to produce a signal indicative of the IOP in the eye. The pluggable proximal section is configured to be connected with and disconnected from the distal section, and comprises: (i) wiring for receiving the signals when connected with the distal section, (ii) a power source, such as battery, and (iii) a wireless communication device configured to transmit the sensed signal to an external system.

The disclosed techniques improve the control of the IOP during phacoemulsification procedures by improving: (i) response time of pressure and temperature sensing, and (ii) control of the fluid flow in the irrigation and aspiration channels of phacoemulsification systems.

### SYSTEM DESCRIPTION

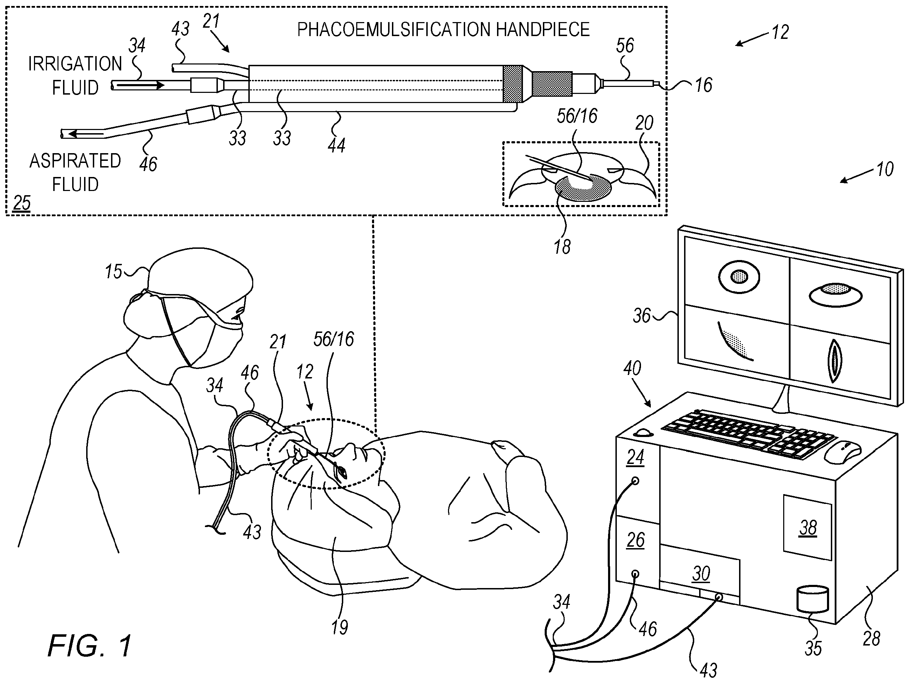

In this section of the description the designation "embodiment" does not imply necessarily that what follows is part of the scope of the claimed invention. The scope of the invention is exclusively defined by the claims. Fig. 1 is a schematic, pictorial illustration of a phacoemulsification system 10, in accordance with an embodiment of the present invention. In some embodiments, system 10 comprises a hand-held device, in the present example a phacoemulsification probe, also referred to herein as a probe 12 for brevity.

Reference is now made to an inset 25 showing details of probe 12. In some embodiments, probe 12 comprises a needle 16 and a coaxial irrigation sleeve 56 that at least partially surrounds needle 16 and creates a fluid pathway between the external wall of the needle and the internal wall of the irrigation sleeve.

In some embodiments, irrigation sleeve 56 may have one or more side ports at or near the distal end, so as to allow irrigation fluid to flow toward the distal end of probe 12 through the fluid pathway and out of the port(s). In such embodiments, the irrigation fluid is configured to cool the distal end of probe 12, and particularly, needle 16, which is heated when one or more piezoelectric crystals (described below) of probe 12 are operating.

Note that needle 16 is hollow, and is configured to aspirate fluids from a patient's eye during a phacoemulsification procedure. In the present example, needle 16 is configured to draw at least eye fluid (e.g., natural eye fluid, irrigation fluid and lens material described below) from the patient's eye to an aspiration channel 44 described below.

In other embodiments, needle 16 or another suitable element of probe 12 may be able to transfer irrigation fluid from an irrigation channel 33 to the patient's eye.

In some embodiments, needle 16 is configured for insertion into a lens capsule 18 of an eye 20 of a patient 19 by a physician 15, so as to remove a cataract. In the present example, needle 16 and irrigation sleeve 56 are shown in inset 25 as a straight object, but in other embodiments any other suitable type of needle may be used with probe 12. For example, a curved or bent tip needle commercially available, for example, from Johnson & Johnson Surgical Vision, Inc., Santa Ana, CA, USA.

Reference is now made back to the general view of Fig. 1. In some embodiments, during the aforementioned phacoemulsification procedure, a pumping sub-system 24 comprised in a console 28 of system 10, is configured to pump irrigation fluid, such as a balanced salt solution (BSS), from an irrigation reservoir (not shown), via an irrigation tube 34 and irrigation channel 33 of probe 12, to irrigation sleeve 56 for irrigating the patient's eye. In other embodiments, pumping sub-system 24 may be coupled with or replaced by a gravity fed irrigation source such as a BSS bottle/bag.

In some embodiments, during the phacoemulsification procedure physician 15 uses the aforementioned piezoelectric crystals of probe 12 to apply vibrations to needle 16 for emulsifying the lens of patient's eye 20. The irrigation fluid is instilled into eye 20 (as described above) during the emulsification, so as to: (i) compensate for eye fluid aspirated from eye 20 (described in detail below) for maintaining constant intraocular pressure (IOP) in eye 20, and (ii) control a constant temperature in eye 20 (e.g., by dissipating heat generated by the vibration of needle 16) during the procedure. Note that during the emulsification of the lens of eye 20, eye fluid comprises natural eye fluid, irrigation fluid, and lens material (e.g., the emulsified parts of the cataract), which is aspirated using probe 12 as described herein.

In some embodiments, the eye fluid is being aspirated, via hollow needle 16 and aspiration channel 44 of probe 12, and via an aspiration tube 46, to a collection receptacle (not shown) using a pumping sub-system 26 of console 28. In such embodiments, aspiration tube 46 is coupled with and running between aspiration channel 44 of probe 12 and pumping sub-system 26 of console 28.

In other embodiments, pumping sub-systems 24 and 26 may have any other suitable configuration in a fluidics cartridge/cassette (not shown) or in console 28 of system 10. For example, both pumping sub-systems 24 and 26 may be co-packaged, and/or at least one of pumping sub-systems 24 and 26 (typically sub-system 24) may use other pumping techniques, such as being coupled with or replaced by the aforementioned gravity fed irrigation source.

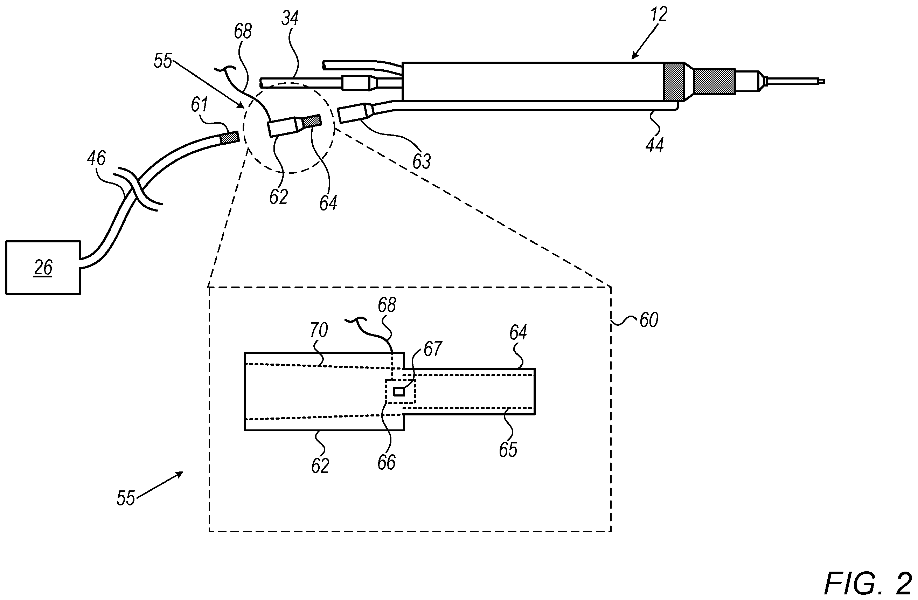

In the example of probe 12 shown in inset 25 of Fig. 1 and also in Figs. 2-5 (discussed below) of the present disclosure, irrigation channel 33 and irrigation tube 34 are aligned with (i.e., facing) needle 16, so as to have direct flow of the irrigation tube into a patient's eye. In this configuration, aspiration channel 44 and aspiration tube 46 are fluidly connected to needle 16 but, as shown in inset 25, are not aligned therewith. In alternative embodiments, probe 12 may have a different configuration in which the positions of the channels (and respective tubes) are exchanged. In such embodiments, aspiration channel 44 and aspiration tube 46 are aligned with needle 16 (instead of the position of irrigation channel 33 and irrigation tube 34 shown in inset 25), and the other way around, such that irrigation channel 33 and irrigation tube 34 are disposed instead of aspiration channel 44 and aspiration tube 46. In other words, irrigation channel 33 and irrigation tube 34 are not aligned with needle 16. In other embodiments, probe 12 may have any other suitable arrangement of the aforementioned channels and respective tubes.

In some embodiments, probe 12 comprises additional elements (not shown), such as but not limited to one or more piezoelectric crystals coupled with a horn for driving vibration of needle 16. The piezoelectric crystal(s) is configured to vibrate needle 16 in a resonant vibration mode. The vibration of needle 16 is used for breaking the aforementioned cataract into small pieces, e.g., constitutes the aforementioned lens material, during the phacoemulsification procedure.

In some embodiments, console 28 comprises a piezoelectric drive module 30, coupled with the one or more piezoelectric crystals, using electrical wiring running in a cable 43. Drive module 30 is controlled by a processor 38 and conveys processor-controlled driving signals via cable 43 to, for example, maintain needle 16 at maximal vibration amplitude. The drive module may be implemented in hardware or software, for example, in a proportionalintegral-derivative (PID) control architecture.

In some embodiments, processor 38 is configured to receive user-based commands via a user interface 40, which may comprise setting a vibration mode and/or frequency of the piezoelectric crystal(s), and setting or adjusting an irrigation and/or aspiration rate of pumping sub-systems 24 and 26. In an embodiment, user interface 40 and a display 36 may be combined as a single touch screen graphical user interface. In an embodiment, physician 15 uses a foot pedal (not shown) as a means of control. Additionally, or alternatively, processor 38 may receive the user-based commands from controls located in a handle 21 of probe 12.

In some embodiments, processor 38 may comprise a general-purpose processor, which is programmed in software to carry out the functions described herein. Some or all of the functions of processor 38 may be combined in a single physical component or, alternatively, implemented using multiple physical components. These physical components may comprise hard-wired or programmable devices, or a combination thereof.

In some embodiments, at least some of the functions of processor 38 may be carried out using suitable software stored in a memory 35. This software may be downloaded to a device in electronic form, over a network, for example. Alternatively, or additionally, the software may be stored in tangible, non-transitory computer-readable storage media, such as optical, magnetic, or electronic memory.

In some embodiments, system 10 and in particular console 28 and probe 12, may comprise additional elements which are omitted for the sake of conceptual clarity. For example, physician 15 typically performs the procedure using a stereo-microscope and/or magnifying glasses and/or augmented/virtual reality head mount display (HMD), neither of which are shown. Physician 15 may use other surgical tools in addition to probe 12, which are also not shown in order to maintain clarity and simplicity of the present disclosure.

This particular configuration of system 10 is shown by way of example, in order to illustrate certain problems that are addressed by embodiments of the present invention and to demonstrate the application of these embodiments in enhancing the performance of such a phacoemulsification system. Embodiments of the present invention, however, are by no means limited to this specific sort of example phacoemulsification system, and the principles described herein may similarly be applied to other sorts of phacoemulsification or other suitable types of surgical systems.

Note that additional components of system 10, and in particular, additional features of probe 12, are shown and descried in detail, for example, in Figs. 2-6 below.

### IMPLEMENTING SENSORS IN OR NEAR A HANDLE OF

### PHACOEMULSIFICATION PROBE

Fig. 2 is a schematic, pictorial illustration of a pressure sensing assembly (PSA) 55 coupled with probe 12, in accordance with an embodiment of the present invention.

In some embodiments, one or more sensors, such as but not limited to pressure sensors and/or temperature sensors, may be coupled with one or both of irrigation tube 34 and aspiration tube 46. In principle, it is also possible to position such pressure and/or temperature sensors (or any other suitable sensors) in close proximity to pumping sub-systems 24 and 26. This approach may be implemented, for example, by positioning the one or more sensors in console 28 or in the aforementioned fluidics cartridge. However, having the sensors at the proximal ends of irrigation tube 34 and aspiration tube 46 may reduce the quality of the phacoemulsification procedure, because changes in the flow rates of the fluids, and especially of the aspirated eye fluid, are typically caused by changes at the distal end of aspiration tube 46 and even further distally, within aspiration channel 44 and the eye being operated on. Thus, positioning the sensors at console 28, may cause a delay in the response time of the sensors to changing pressure and/or temperature in close proximity to the patient's eye. Moreover, even when responding to a change in sensed pressure, the pressure sensed in response to a change in pressure (e.g., vacuum) applied to aspiration tube 46 by pumping sub-system 26, may not be indicative of the actual vacuum applied to the patient's eye by aspiration channel 44, because the length of aspiration tube 46 (e.g., about 2 meters) acts as a "dead space" for the sensing and closedloop control of the aspiration pressure.

Therefore, in order to receive measurements indicative of the actual pressure or temperature in close proximity to the patient's eye, it is important to perform the sensing as close as possible to the patient's eye.

In some embodiments, the pressure and/or temperature and/or other sensors may be disposed in close proximity to one or both of irrigation channel 33 and aspiration channel 44. Such sensors are configured to produce electrical signals, such as pressure and temperature signals indicative, respectively, of pressure and temperature of one or both of the irrigation fluid flowing in irrigation channel 33, and the eye fluid flowing in aspiration channel 44, as described in Fig. 1 above.

In some embodiments, based on the electrical signals described above, processor 38 is configured to control, for example, the pressure of the irrigation fluid and/or eye fluid by adjusting, respectively, the irrigation and/or aspiration rate of pumping sub-systems 24 and 26.

In the present example, PSA 55 is coupled between aspiration tube 46 and aspiration channel 44 and is configured to sense the pressure of the eye fluid flowing between aspiration channel 44 and aspiration tube 46.

In some embodiments, PSA 55 comprises a hollow tube 65, which is inserted between aspiration channel 44 and aspiration tube 46, and is configured to flow eye fluid from the patient's eye to a collection reservoir.

In some embodiments, PSA 55 comprises a pressure sensing device (PSD) 66, which is incorporated into a flexible printed circuit board (PCB) 70 wrapped around and coupled with hollow tube 65 using any suitable coupling technique.

In some embodiments, hollow tube 65 may have an opening 67 so that PSD 66, which is bonded to opening 67, is exposed to the flowing eye fluid for measuring the fluid pressure thereof, and also blocks the eye fluid from leaking through opening 67. In other embodiments, PSD 66 may be positioned within hollow tube 65, and therefore makes direct contact with the eye fluid. In both embodiments, PSD 66 is placed in direct contact with the eye fluid and is configured to produce a pressure signal indicative of the pressure of the eye fluid, which is extracted from the eye, and is flowing between aspiration channel 44 and aspiration tube 46.

In some embodiments, hollow tube 65 comprises, a first luer connector coupled with aspiration channel 44, and a second luer connector coupled with aspiration tube 46. In the present example, hollow tube 65 comprises: (i) a female luer 62 coupled with a male luer 61 of aspiration tube 46, and (ii) a male luer 64 coupled with a female luer 63 of aspiration channel 44. In other embodiments, hollow tube 65 may be coupled between aspiration channel 44 and aspiration tube 46 using any other suitable coupling technique. Moreover, in the example of Fig. 2, PSD 66 is positioned between luers 62 and 64 of hollow tube 65, but in other embodiments, PSD 66 may be placed at any other suitable position within or on hollow tube 65 allowing direct contact between PSD 66 and the eye fluid flowing through hollow tube 65 toward pumping sub-system 26 of console 28.

In some embodiments, PSA 55 may comprise a reusable assembly or a disposal assembly. Additionally, or alternatively, a temperature sensing device (not shown) may be placed in contact with the eye fluid so as to measure the temperature thereof. For example, hollow tube 65 may have an additional opening, and the temperature sensing device may be incorporated into flexible PCB 70 and coupled with the additional opening using the same techniques applied to PSD 66. In some embodiments, flexible PCB 70 may be electrically connected to processor 38 via an electrical wire 68, which may be connected to cable 43 (shown in Fig. 1 above) or any other suitable cable (not shown) of system 10, connected to processor 38.

In some embodiments, PSA 55 may be produced by forming and coupling flexible PCB 70 with hollow tube 65, such that PSD 66 is exposed to the flowing fluid and blocks opening 67. Subsequently, male luer 64 of PSA 55 is fitted into female luer 63 of aspiration channel 44, and female luer 62 of PSA 55 is fitted over male luer 61 of aspiration tube 46.

This particular configuration of PSA 55 is shown by way of example, in order to illustrate certain problems that are addressed by embodiments of the present invention and to demonstrate the application of these embodiments in enhancing the performance of such a phacoemulsification system. Embodiments of the present invention, however, are by no means limited to this specific sort of example sensing assembly, and the principles described herein may similarly be applied to other sorts of sensing assemblies used in other sorts of phacoemulsification systems or other systems configured to flow fluids.

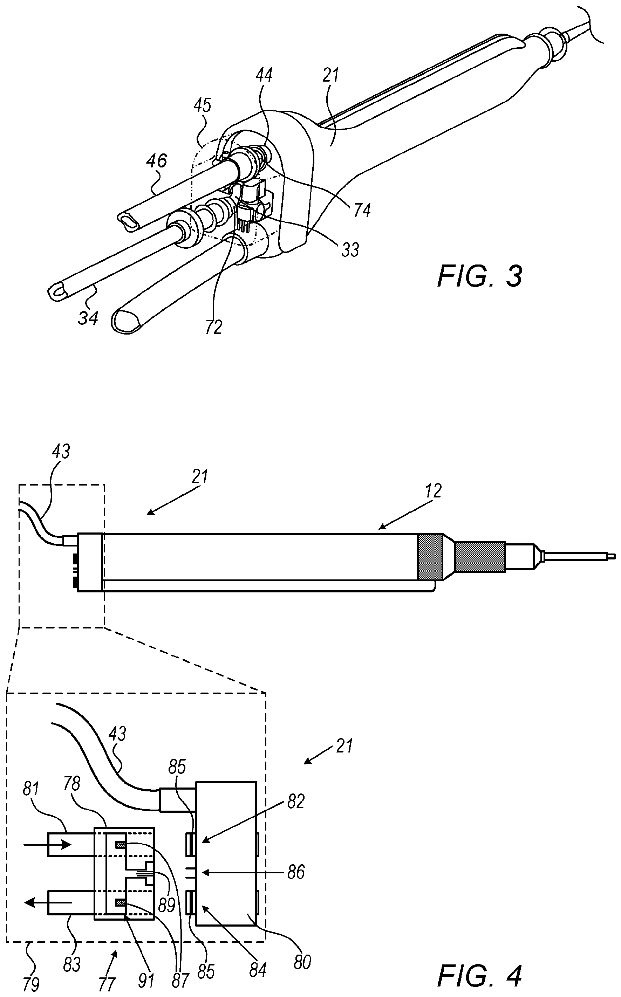

Fig. 3 is a schematic, pictorial illustration of sensors 72 and 74 disposed on handle 21 at the proximal end of probe 12, in accordance with another embodiment of the present invention. In the example of Fig. 3, sensors 72 and 74 are coupled with the proximal end of handle 21.

In some embodiments, sensor 72 is disposed between irrigation channel 33 and irrigation tube 34, and is configured to measure the fluid pressure of the irrigation fluid flowing into irrigation channel 33 of probe 12.

In some embodiments, sensor 74 is disposed between aspiration channel 44 and aspiration tube 46, and is configured to measure the fluid pressure of the eye fluid flowing from aspiration channel 44 into aspiration tube 46. Note that in the aspiration channel, the eye fluid is extracted from the patient's eye by applying vacuum to aspiration tube 46 and aspiration channel 44.

The position of aspiration channel 44 and irrigation channel 33 are shown by way of example. In other embodiments, the positions may be flipped so that sensor 72 may measure the eye fluid pressure flowing in aspiration channel 44, and sensor 74 may measure the irrigation fluid pressure flowing in irrigation channel 33.

In some embodiments, handle 21 comprises a case 45, which is configured to cover over sensors 72 and 74, so as to protect the sensors from the surrounding environment, e.g., from being wetted or mechanically damaged.

In some embodiments, sensors 72 and 74 may be disposed in close proximity to irrigation channel 33 and aspiration channel 44, respectively. In other embodiments, sensors 72 and 74, and optionally additional sensors, may be incorporated in an assembly coupled with handle 21.

Fig. 4 is a schematic, pictorial illustration of a connector assembly 77 of a phacoemulsification system, in accordance with another embodiment of the present invention. Connector assembly 77 may be implemented, for example, with handle 21 of phacoemulsification system 10 of Fig. 1 above.

Reference is now made to an inset 79 showing connector assembly 77. In some embodiments, connector assembly 77 is configured to connect with an adaptor 80 disposed at the proximal end of handle 21 of probe 12. In the present example, adaptor 80 comprises one or more electrical pins 86, inlet 82, and outlet 84 of irrigation channel 33 and aspiration channel 44, respectively. As shown in inset 79, inlet 82 and outlet 84 have O-rings 85 configured to seal irrigation channel 33 and aspiration channel 44 from leaks of the irrigation and eye fluids during the operation of system 10.

In some embodiments, connector assembly 77 comprises a housing 78 comprising an irrigation passage 81, which is connecting between irrigation channel 33 and irrigation tube 34, and is configured to pass the irrigation fluid from irrigation tube 34 into irrigation channel 33.

In some embodiments, housing 78 comprises an aspiration passage 83, which is connecting between aspiration channel 44 and aspiration tube 46, and is configured to pass the eye fluid (typically with the lens material) from aspiration channel 44 to console 28, via aspiration tube 46.

In some embodiments, housing 78 contains one or more pressure and/or temperature sensors 87, which are incorporated into a flexible printed circuit board (PCB) 91 or into any other suitable substrate or connecting scheme. Sensors 87 are configured for sensing the fluid pressure and/or the fluid temperature, at one of, and typically, as shown in Fig. 4, at both of irrigation channel 33 and aspiration channel 44.

In some embodiments, PCB 91 of housing 78 comprises an electrical connector 89, e.g., a socket, which is configured to mate with, and separate from, pins 86 for connecting and disconnecting, respectively, between housing 78 of connector 77 and adaptor 80 of handle 21 of probe 12.

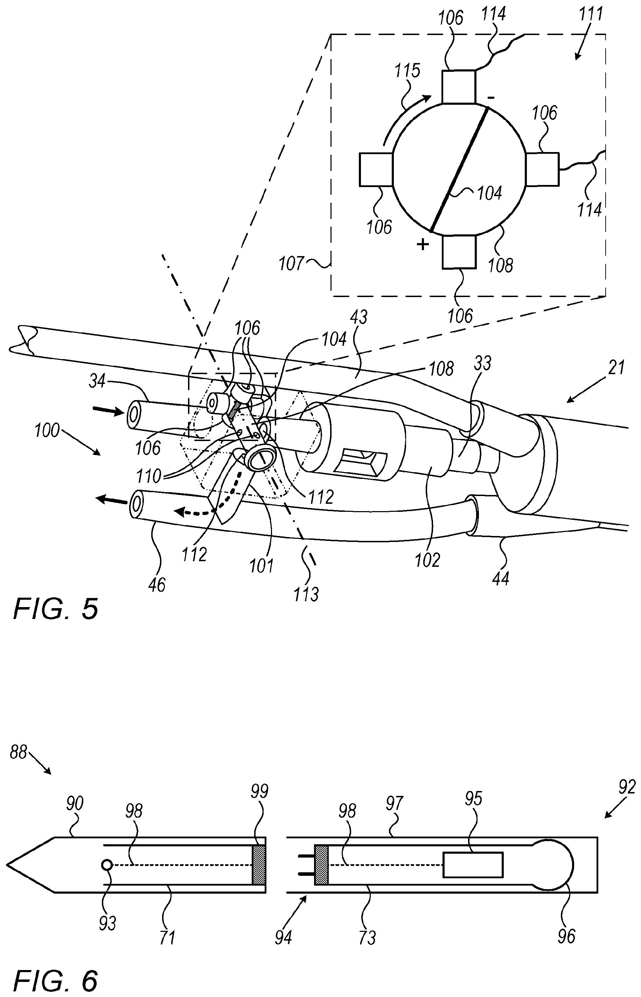

In some embodiments, when disconnected from adaptor 80, electrical connector 77 is configured to disable the operation of probe 12 so as to prevent emulsification of the eye lens without controlled irrigation and aspiration as described above. In other embodiments, housing 78 of electrical connector 77 may contain one or more valves (not shown), which is controlled by processor 38 and is configured to control the flow level of one or both of the irrigation and eye fluids in response to pressure and/or temperature signals received from sensors 87. The valves may comprise at least a linear valve or a rotating valve, such as the valve described in Fig. 5 below.

In some embodiments, electrical connector 77 may be produced by: (i) providing housing 78 having passages 81 and 83, and (ii) inserting flexible PCB 91, having sensors 87 and 89, and connector 89, into housing 78. Electrical connector 77 may be connected with adaptor 80 by: (i) fitting housing 78 over inlet 82 and outlet 84, and (ii) fitting connector 89 of electrical connector 77 over pins 86 of adaptor 80.

This particular configuration of connector 77 and adaptor 80 are shown by way of example, in order to illustrate certain problems that are addressed by embodiments of the present invention and to demonstrate the application of these embodiments in enhancing the performance of such a phacoemulsification system. Embodiments of the present invention, however, are by no means limited to this specific sort of example connector and adaptor, and the principles described herein may similarly be applied to other sorts of connecting scheme used in system 10 or is other sorts of phacoemulsification and other systems.

### ADJUSTING ASPIRATION PRESSURE DURING PHACOEMULSIFICATION PROCEDURES

Fig. 5 is a schematic, pictorial illustration of a rotatable valve 100 incorporated into or coupled with handle 21 of probe 12, in accordance with another embodiment of the present invention.

In some embodiments, valve 100 comprises a solid tube 108 having multiple openings 110 configured to pass fluids through inlets/outlets 112 as will be described in detail below. In the present example, valve 100 is disposed between irrigation channel 33, irrigation tube 34 and aspiration tube 46 (via an aspiration bypass 101 as described below), but in other embodiments, valve 100 may be disposed between irrigation tube 34 and aspiration tube 46, or using any other suitable configuration.

In some embodiments, valve 100 is configured to rotate for toggling between a first position (e.g., when at least one of aspiration channel 44 and aspiration tube 46 is at least partially obstructed by lens material) and a second position (e.g., when both aspiration channel 44 and aspiration tube 46 are unobstructed). In the first position, valve 100 is configured to enable flow of at least part of the irrigation fluid, through aspiration bypass 101, into aspiration tube 46, and optionally at the same time, block passage of at least part of the irrigation fluid into irrigation channel 33.

In the second position, valve 100 is configured to: (i) block passage of the irrigation fluid, into aspiration bypass 101 and aspiration tube 46, by having tube 108 rotated so that openings 110 are not facing inlets/outlets 112, and at the same time, (ii) allow passage of the irrigation fluid into irrigation channel 33, so as to flow the irrigation fluid into the patient's eye. Note that in both positions, the irrigation fluid flows via tube 108, through one or more openings 110 and one or more inlets/outlets 112.

As described above, maintaining constant IOP in the patient's eye is critical during a phacoemulsification procedure. Therefore, toggling between enabling and blocking the flow of the irrigation fluid into aspiration channel 44 (in response to the sensed pressure described above) has to be carried out very fast, for example, within about one millisecond.

Reference is now made to an inset 107 showing a rotating apparatus (RA) 111 of valve 100. In some embodiments, RA 111 comprises multiple solenoids 106, wrapped around, surrounding, or otherwise coupled with tube 108. In some embodiments, RA 111 comprises one or more shafts 104 (in the present example one shaft 104), made from any suitable magnetic material (e.g., ferrite) for serving as a magnet, and coupled with tube 108, e.g., across the diameter thereof. In this configuration, shaft 104 and tube 108 constitute one rigid part, so that when shaft 104 rotates, tube 108 rotates therewith. Note that solenoids 106 are electrically connected to a power supply (not shown) via electrical wires 114.

In some embodiments, processor 38 is configured to control the power supply to apply suitable electrical current to solenoids 106, so as to rotate shaft 104, which is magnetic and therefore has an electric dipole. In such embodiments, in response to the electrical current applied to solenoids 106, tube 108 and shaft 104 are rotating together about an axis 113 (shown in the general view of Fig. 5), e.g., in a direction 115, so as to enable or block the aforementioned flow of the irrigation fluid into aspiration channel 44 or aspiration tube 46 as described above. Note that RA 111 is configured to toggle between the first and second positions within a fraction of a millisecond, and therefore to maintain the required constant IOP in the patient's eye during the phacoemulsification procedure.

In other embodiments, valve 100 may have additional openings and/or additional sets of solenoids 106 and shaft 104, so as to have additional positions other than the aforementioned first and second positions for directing any fluids between any two or more tubes or channels of system 10.

In some embodiments, valve 100 may be produced by: (i) producing RA 111 by inserting shaft 104 across the diameter of tube 108 having openings 110. Shaft 104 is typically (but not necessarily) positioned orthogonal to axis 113, (ii) solenoids 106 are coupled with or wrapped around the outer surface of tube 108 as described above, and are electrically connected (e.g., wired) to the power supply described above, and (iii) valve 100 is coupled between irrigation channel 33 and aspiration channel 44, or between irrigation tube 34 and aspiration tube 46, or using any other suitable connecting scheme to enable flowing irrigation fluid into aspiration channel 44. Note that the coupling may be carried out directly, or using an irrigation adaptor 102.

### WIRELESS DEVICE FOR MEASURING INTRAOCULAR PRESSURE

Fig. 6 is a schematic, pictorial illustration of an ophthalmic handpiece (OHP) 88 configured for measuring intraocular pressure (IOP) in a patient's eye, in accordance with another embodiment of the present invention. In some embodiments, OHP 88 may be coupled with or designed into probe 12, or may be used as a stand-alone handpiece, for measuring pressure before, during and after a phacoemulsification procedure.

In some embodiments, OHP 88 comprises a distal section 90, which is typically disposable, and a proximal section 92, which is typically reusable. Distal section 90 is configured to be inserted into the eye in question and comprises a substrate, such as a PCB 71 having electrical traces 98. In some embodiments, a pressure sensor 93 and a connector 99 are coupled with PCB 71 and are connected to one another via electrical traces 98. Pressure sensor 93 in configured to produce a signal indicative of the IOP in the eye, and connector 99 is configured for connecting between distal section 90 and proximal section 92.

In some embodiments, proximal section 92 comprises a substrate, such as a PCB 73 having electrical traces 98 and configured for incorporating a connector 94 and multiple active devices, such as a battery 96 or any other suitable type of power source, and a wireless communication device 95 configured to transmit the sensed signal to processor 38 of system 10.

In some embodiments, electrical traces 98 are configured for connecting between connector 94 and the aforementioned active devices, for receiving the signals produced by pressure sensor 93 when distal section 90 and proximal section 92 are connected by connectors 99 and 94.

In some embodiments, OHP 88 comprises a sterile sleeve 97, which is wrapped around and configured to seal proximal section 92, so as to enable reuse of proximal section 92 with additional distal sections 90 during additional measurements of IOP, e.g., in eyes of other patients, without sanitizing proximal section 92.

The configuration of OHP 88 is provided by way of example, and is simplified for the sake of conceptual clarity. For example, OHP 88 may comprise a memory device (not shown) for storing the measurements received from pressure sensor 93 and control circuitry (not shown) for controlling the memory, wireless communication device 95 and other components of OHP 88.

In some embodiments, OHP 88 may be produced by producing: (i) distal section 90 for insertion into a patient's eye, and (ii) proximal section 92 for transmitting the signals received from the aforementioned pressure sensor 93. Distal section 90 may be produced by coupling pressure sensor 93 and connector 99 with electrical traces 98 at predefined locations of PCB 71, and coupling PCB 71 to the housing of distal section 90.

In some embodiments, proximal section 92 may be produced by (i) coupling with electrical traces 98 of PCB 73: connector 94, wireless communication device 95, battery 96 and any other components, such as but not limited to the aforementioned memory device and control circuitry, (ii) coupling PCB 73 with the housing of proximal section 92, and (iii) sealing proximal section 92 using sterile sleeve 97. Note that when measuring IOP, proximal section 92 is connected to distal section using connectors 94 and 99.

Although the embodiments described herein mainly address phacoemulsification systems, the methods and systems described herein can also be used in other applications.

It will thus be appreciated that the embodiments described above are cited by way of example, and that the present invention is not limited to what has been particularly shown and described hereinabove. ,

## Claims

1. A phacoemulsification system (10), comprising:
a phacoemulsification probe (12), comprising:
a needle (16), which is configured to be inserted into a lens capsule of an eye and to be vibrated to emulsify a lens of the eye;
an irrigation channel (33), configured for flowing irrigation fluid into the lens capsule; and
an aspiration channel (44), configured for removing at least eye fluid from the lens capsule; and **characterized by**
a sensing assembly (55), which is configured to be coupled with a proximal end of the phacoemulsification probe and is configured to sense a pressure of the eye fluid, wherein the sensing assembly comprises a pressure sensing assembly, PSA, configured to be coupled between an aspiration tube (46) and the aspiration channel and configured to sense the pressure of the eye fluid flowing between the aspiration channel and the aspiration tube, wherein the PSA comprises: (i) a hollow tube (65), which is inserted between the aspiration channel and the aspiration tube, and is configured to permit flow of at least the eye fluid, and (ii) a pressure sensing device, PSD (66), which is placed in direct contact with the eye fluid flowing in the hollow tube and is configured to produce a pressure signal indicative of the pressure of the eye fluid flowing between the aspiration channel and the aspiration tube, wherein the hollow tube comprises, a first connector (64) coupled with the aspiration channel, and a second connector (62)coupled with the aspiration tube, and wherein the PSD is positioned between the first connector and the second connector.

2. The phacoemulsification system according to claim 1, further comprising a wire (68), which is connected to the PSD and is configured to transmit the pressure signal to a processor of a phacoemulsification console.

3. The phacoemulsification system according to claim 1, wherein the sensing assembly comprises a temperature sensor coupled with the aspiration channel and configured to sense a temperature of the eye fluid at the proximal end of the phacoemulsification probe.

4. A phacoemulsification system (10), comprising:
a phacoemulsification probe (12), comprising:
a needle (16), which is configured to be inserted into a lens capsule of an eye and to be vibrated to emulsify a lens of the eye;
an irrigation channel (33), configured for flowing irrigation fluid into the lens capsule; and
an aspiration channel (44), configured for removing at least eye fluid from the lens capsule; and **characterized by**
a sensing assembly (55), which is configured to be coupled with a proximal end of the phacoemulsification probe and is configured to sense a pressure of the irrigation fluid, wherein the sensing assembly comprises a pressure sensing assembly, PSA, coupled between an irrigation tube (34) and the irrigation channel and configured to sense the pressure of the irrigation fluid flowing between the irrigation tube and the irrigation channel, wherein the PSA comprises: (i) a hollow tube (65), which is inserted between the irrigation channel and the irrigation tube, and is configured to permit flow of at least the irrigation fluid, and (ii) a pressure sensing device, PSD (67), which is placed in direct contact with the irrigation fluid flowing in the hollow tube and is configured to produce a pressure signal indicative of the pressure of the irrigation fluid flowing between the irrigation tube and the irrigation channel, and wherein the hollow tube comprises, a first luer connector (64) coupled with the irrigation channel, and a second luer connector (62) coupled with the irrigation tube, and wherein the PSD is positioned between the first luer connector and the second luer connector.

5. The phacoemulsification system according to claim 1 wherein the first connector is a first luer connector, and the second connector is a second luer connector.

6. The phacoemulsification system according to claim 4, further comprising a wire (68), which is connected to the PSD and is configured to transmit the pressure signal to a processor of a phacoemulsification console.

7. The phacoemulsification system according to claim 1, wherein the sensing assembly further comprises a second sensing assembly, wherein the second assembly comprises a second pressure sensing assembly, PSA, coupled between an irrigation tube and the irrigation channel and configured to sense the pressure of the irrigation fluid flowing between the irrigation tube and the irrigation channel.

8. The phacoemulsification system according to claim 7, wherein the second PSA comprises: (i) a second hollow tube, which is inserted between the irrigation channel and the irrigation tube, and is configured to permit flow of at least the irrigation fluid, and (ii) a second pressure sensing device, PSD, which is placed in direct contact with the irrigation fluid flowing in the second hollow tube and is configured to produce a pressure signal indicative of the pressure of the irrigation fluid flowing between the irrigation tube and the irrigation channel.

9. The phacoemulsification system according to claim 8, wherein the second hollow tube comprises a third connector coupled with the irrigation channel, and a fourth connector coupled with the irrigation tube, and wherein the second PSD is positioned between the third connector and the fourth connector, optionally wherein the third connector is a third luer connector, and the fourth connector is a fourth luer connector.

10. A phacoemulsification system (10), comprising:
a phacoemulsification probe (12), comprising:
a needle (16), which is configured to be inserted into a lens capsule of an eye and to be vibrated to emulsify a lens of the eye;
an irrigation channel for flowing irrigation fluid into the lens capsule; and
an aspiration channel for removing at least eye fluid from the lens capsule; and **characterized by** a connector assembly (80), comprising:
a housing, comprising: (i) an irrigation passage (81), for connecting between the irrigation channel (33) and an irrigation tube (34) and configured to pass the irrigation fluid into the irrigation channel, and (ii) an aspiration passage (83), for connecting between the aspiration channel (44) and an aspiration tube (46) and configured to pass at least the eye fluid away from the aspiration channel to the aspiration tube; and
one or more sensors (87), contained in the housing and configured to measure at least one of fluid pressure and fluid temperature at one or both of the irrigation channel and the aspiration channel.

11. The phacoemulsification system according to claim 10, further comprising an electrical connector (86), which is configured to connect and disconnect the connector assembly and the phacoemulsification probe, and wherein, when the connector assembly is disconnected from the phacoemulsification probe, operation of the phacoemulsification probe is disabled.

12. The phacoemulsification system according to claim 10, wherein the one or more sensors comprise a first pressure sensor disposed in the irrigation passage and a second pressure sensor disposed in the aspiration passage.

13. The phacoemulsification system according to claim 10, further comprising one or more valves (100), wherein the one or more valves are coupled with the irrigation passage or the aspiration passage and configured to control flow of the eye fluid or irrigation fluid.

14. A method for producing a connector assembly, the method comprising:
receiving a housing, comprising: (i) an irrigation passage, connecting between an irrigation channel of a phacoemulsification probe and an irrigation tube for passing irrigation fluid into the irrigation channel, and (ii) an aspiration passage, connecting between an aspiration channel and an aspiration tube for passing at least an eye fluid away from the aspiration channel to the aspiration tube; and
disposing, in the housing, one or more sensors for measuring at least one of fluid pressure and fluid temperature at one or both of the irrigation channel and the aspiration channel.

15. The method according to claim 14, further comprising:
coupling with the housing, an electrical connector, for connecting and disconnecting the connector assembly and the phacoemulsification probe, and wherein, when the connector assembly is disconnected from the phacoemulsification probe, operation of the phacoemulsification probe is disabled, optionally wherein disposing the one or more sensors comprises disposing: (i) a first pressure sensor in the irrigation passage, and (ii) a second pressure sensor in the aspiration passage.

## Patentansprüche

1. Phakoemulsifikationssystem (10), umfassend:
eine Phakoemulsifikationssonde (12), umfassend:
eine Nadel (16), die konfiguriert ist, um in eine Linsenkapsel eines Auges eingeführt zu werden und in Vibration versetzt zu werden, um eine Linse des Auges zu emulgieren;
einen Spülkanal (33), der zum Einströmen von Spülfluid in die Linsenkapsel konfiguriert ist; und
einen Absaugkanal (44), der zum Entfernen von mindestens Augenfluid aus der Linsenkapsel konfiguriert ist; und **gekennzeichnet durch**
eine Erfassungsanordnung (55), die konfiguriert ist, um mit einem proximalen Ende der Phakoemulsifikationssonde gekoppelt zu werden, und konfiguriert ist, um einen Druck des Augenfluids zu erfassen, wobei die Erfassungsanordnung eine Druckerfassungsanordnung, PSA, umfasst, die konfiguriert ist, um zwischen einem Absaugschlauch (46) und dem Absaugkanal gekoppelt zu werden, und konfiguriert ist, um den Druck des Augenfluids zu erfassen, der zwischen dem Absaugkanal und dem Absaugschlauch fließt, wobei die PSA umfasst: (i) ein Hohlrohr (65), das zwischen den Absaugkanal und dem Absaugschlauch eingesetzt ist und konfiguriert ist, um einen Fluss von mindestens dem Augenfluid zu ermöglichen, und (ii) eine Druckerfassungsvorrichtung, PSD (66), die in direktem Kontakt mit dem in dem Hohlrohr fließenden Augenfluid platziert ist und konfiguriert ist, um ein Drucksignal zu erzeugen, das den Druck des zwischen dem Absaugkanal und dem Absaugschlauch fließenden Augenfluid anzeigt, wobei das Hohlrohr einen ersten Verbinder (64), der mit dem Absaugkanal gekoppelt ist, und einen zweiten Verbinder (62), der mit dem Absaugschlauch gekoppelt ist, umfasst und wobei die PSD zwischen dem ersten Verbinder und dem zweiten Verbinder positioniert ist.

2. Phakoemulsifikationssystem nach Anspruch 1, ferner umfassend einen Draht (68), der mit der PSD verbunden ist und konfiguriert ist, um das Drucksignal an einen Prozessor einer Phakoemulsifikationskonsole zu übertragen.

3. Phakoemulsifikationssystem nach Anspruch 1, wobei die Erfassungsanordnung einen Temperatursensor umfasst, der mit dem Absaugkanal gekoppelt ist und konfiguriert ist, um eine Temperatur des Augenfluids an dem proximalen Ende der Phakoemulsifikationssonde zu erfassen.

4. Phakoemulsifikationssystem (10), umfassend:
eine Phakoemulsifikationssonde (12), umfassend:
eine Nadel (16), die konfiguriert ist, um in eine Linsenkapsel eines Auges eingeführt zu werden und in Vibration versetzt zu werden, um eine Linse des Auges zu emulgieren;
einen Spülkanal (33), der zum Einströmen von Spülfluid in die Linsenkapsel konfiguriert ist; und
einen Absaugkanal (44), der zum Entfernen von mindestens Augenfluid aus der Linsenkapsel konfiguriert ist; und **gekennzeichnet durch**
eine Erfassungsanordnung (55), die konfiguriert ist, um mit einem proximalen Ende der Phakoemulsifikationssonde gekoppelt zu werden und konfiguriert ist, um einen Druck des Spülfluids zu erfassen, wobei die Erfassungsanordnung eine Druckerfassungsanordnung, PSA, umfasst, die zwischen einem Spülschlauch (34) und dem Spülkanal gekoppelt ist und konfiguriert ist, um den Druck des Spülfluids zu erfassen, die zwischen dem Spülschlauch und dem Spülkanal fließt, wobei die PSA umfasst: (i) ein Hohlrohr (65), das zwischen dem Spülkanal und dem Spülschlauch eingefügt ist und konfiguriert ist, um einen Fluss von mindestens dem Spülfluid zu ermöglichen, und (ii) eine Druckerfassungsvorrichtung, PSD (67), die in direktem Kontakt mit dem in dem Hohlrohr fließenden Spülfluid platziert ist und konfiguriert ist, um ein Drucksignal zu erzeugen, das den Druck des zwischen dem Spülschlauch und dem Spülkanal fließenden Spülfluids anzeigt, und wobei das Hohlrohr einen ersten Luer-Verbinder (64), der mit dem Spülkanal gekoppelt ist, und einen zweiten Luer-Verbinder (62), der mit dem Spülschlauch gekoppelt ist, umfasst und wobei die PSD zwischen dem ersten Luer-Verbinder und dem zweiten Luer-Verbinder positioniert ist.

5. Phakoemulsifikationssystem nach Anspruch 1, wobei der erste Verbinder ein erster Luer-Verbinder ist und der zweite Verbinder ein zweiter Luer-Verbinder ist.

6. Phakoemulsifikationssystem nach Anspruch 4, ferner umfassend einen Draht (68), der mit der PSD verbunden ist und konfiguriert ist, um das Drucksignal an einen Prozessor einer Phakoemulsifikationskonsole zu übertragen.

7. Phakoemulsifikationssystem nach Anspruch 1, wobei die Erfassungsanordnung ferner eine zweite Erfassungsanordnung umfasst, wobei die zweite Anordnung eine zweite Druckerfassungsanordnung, PSA, umfasst, die zwischen einem Spülschlauch und dem Spülkanal gekoppelt ist und konfiguriert ist, um den Druck des Spülfluids zu erfassen, das zwischen dem Spülschlauch und dem Spülkanal fließt.

8. Phakoemulsifikationssystem nach Anspruch 7, wobei die zweite PSA umfasst: (i) ein zweites Hohlrohr, das zwischen dem Spülkanal und dem Spülschlauch eingefügt ist und konfiguriert ist, um einen Fluss von mindestens dem Spülfluid zu ermöglichen, und (ii) eine zweite Druckerfassungsvorrichtung, PSD, die in direktem Kontakt mit dem in dem zweiten Hohlrohr fließenden Spülfluid platziert ist und konfiguriert ist, um ein Drucksignal zu erzeugen, das den Druck des zwischen dem Spülschlauch und dem Spülkanal fließenden Spülfluids anzeigt.

9. Phakoemulsifikationssystem nach Anspruch 8, wobei das zweite Hohlrohr einen dritten Verbinder, der mit dem Spülkanal gekoppelt ist, und einen vierten Verbinder, der mit dem Spülschlauch gekoppelt ist, umfasst, und wobei die zweite PSD zwischen dem dritten Verbinder und dem vierten Verbinder positioniert ist, optional wobei der dritte Verbinder ein dritter Luer-Verbinder ist und der vierte Verbinder ein vierter Luer-Verbinder ist.

10. Phakoemulsifikationssystem (10), umfassend:
eine Phakoemulsifikationssonde (12), umfassend:
eine Nadel (16), die konfiguriert ist, um in eine Linsenkapsel eines Auges eingeführt zu werden und in Vibration versetzt zu werden, um eine Linse des Auges zu emulgieren;
einen Spülkanal zum Einströmen von Spülfluid in die Linsenkapsel; und
einen Absaugkanal zum Entfernen von mindestens Augenfluid aus der Linsenkapsel; und **gekennzeichnet durch** eine Verbinderanordnung (80), umfassend:
ein Gehäuse, umfassend: (i) einen Spüldurchgang (81) zum Verbinden zwischen dem Spülkanal (33) und einem Spülschlauch (34) und konfiguriert zum Leiten des Spülfluids in den Spülkanal, und (ii) einen Absaugdurchgang (83) zum Verbinden zwischen dem Absaugkanal (44) und einem Absaugschlauch (46) und konfiguriert zum Leiten mindestens des Augenfluids weg von dem Absaugkanal zu dem Absaugschlauch; und
einen oder mehrere Sensoren (87), die in dem Gehäuse enthalten und konfiguriert sind, um mindestens eines von Fluiddruck und Fluidtemperatur an einem oder beiden von dem Spülkanal und dem Absaugkanal zu messen.

11. Phakoemulsifikationssystem nach Anspruch 10, ferner umfassend einen elektrischen Verbinder (86), der konfiguriert ist, um die Verbinderanordnung und die Phakoemulsifikationssonde zu verbinden und zu trennen, und wobei, wenn die Verbinderanordnung von der Phakoemulsifikationssonde getrennt ist, der Betrieb der Phakoemulsifikationssonde deaktiviert ist.

12. Phakoemulsifikationssystem nach Anspruch 10, wobei der eine oder die mehreren Sensoren einen ersten Drucksensor, der in dem Spüldurchgang angeordnet ist, und einen zweiten Drucksensor, der in dem Absaugdurchgang angeordnet ist, umfassen.

13. Phakoemulsifikationssystem nach Anspruch 10, ferner umfassend ein oder mehrere Ventile (100), wobei das eine oder die mehreren Ventile mit dem Spüldurchgang oder dem Absaugkanal gekoppelt sind und konfiguriert sind, um den Fluss des Augenfluids oder Spülfluids zu steuern.

14. Verfahren zum Produzieren einer Verbinderanordnung, das Verfahren umfassend:
Aufnehmen eines Gehäuses, umfassend: (i) einen Spüldurchgang, der zwischen einem Spülkanal einer Phakoemulsifikationssonde und einem Spülschlauch zum Leiten von Spülfluid in den Spülkanal verbindet, und (ii) einen Absaugdurchgang, der zwischen einem Absaugkanal und einem Absaugschlauch zum Leiten mindestens eines Augenfluids weg von dem Absaugkanal zu dem Absaugschlauch verbindet; und
Anordnen, in dem Gehäuse, eines oder mehrerer Sensoren zum Messen mindestens eines von Fluiddruck und Fluidtemperatur an einem oder beiden von dem Spülkanal und dem Absaugkanal.

15. Verfahren nach Anspruch 14, ferner umfassend:
Koppeln eines elektrischen Verbinders mit dem Gehäuse zum Verbinden und Trennen der Verbinderanordnung und der Phakoemulsifikationssonde, und wobei, wenn die Verbinderanordnung von der Phakoemulsifikationssonde getrennt ist, der Betrieb der Phakoemulsifikationssonde deaktiviert ist, optional wobei das Anordnen des einen oder der mehreren Sensoren das Anordnen umfasst: (i) einen ersten Drucksensor in dem Spüldurchgang und (ii) einen zweiten Drucksensor in dem Absaugdurchgang.

## Revendications

1. Système de phacoémulsification (10), comprenant :
une sonde de phacoémulsification (12), comprenant :
une aiguille (16), qui est conçue pour être insérée dans une capsule cristallinienne d'un œil et pour être mise en vibration pour émulsifier un cristallin de l'œil ;
un canal d'irrigation (33), conçu pour l'écoulement d'un fluide d'irrigation dans la capsule cristallinienne ; et
un canal d'aspiration (44), conçu pour retirer au moins un fluide oculaire de la capsule cristallinienne ; et **caractérisé par**
un ensemble de captage (55), qui est conçu pour être accouplé à une extrémité proximale de la sonde de phacoémulsification et est configuré pour capter une pression du fluide oculaire, dans lequel l'ensemble de captage comprend un ensemble de captage de pression, PSA, conçu pour être accouplé entre un tube d'aspiration (46) et le canal d'aspiration et configuré pour capter la pression du fluide oculaire s'écoulant entre le canal d'aspiration et le tube d'aspiration, dans lequel le PSA comprend : (i) un tube creux (65), qui est inséré entre le canal d'aspiration et le tube d'aspiration, et est conçu pour permettre un écoulement au moins du fluide oculaire, et (ii) un dispositif de captage de pression, PSD (66), qui est placé en contact direct avec le fluide oculaire s'écoulant dans le tube creux et est configuré pour produire un signal de pression indiquant la pression du fluide oculaire s'écoulant entre le canal d'aspiration et le tube d'aspiration, dans lequel le tube creux comprend, un premier raccord (64) accouplé au canal d'aspiration, et un deuxième raccord (62) accouplé au tube d'aspiration, et dans lequel le PSD est positionné entre le premier raccord et le deuxième raccord.

2. Système de phacoémulsification selon la revendication 1, comprenant en outre un fil (68), qui est connecté au PSD et est configuré pour transmettre le signal de pression à un processeur d'une console de phacoémulsification.

3. Système de phacoémulsification selon la revendication 1, dans lequel l'ensemble de captage comprend un capteur de température accouplé au canal d'aspiration et configuré pour capter une température du fluide oculaire au niveau de l'extrémité proximale de la sonde de phacoémulsification.

4. Système de phacoémulsification (10), comprenant :
une sonde de phacoémulsification (12), comprenant :
une aiguille (16), qui est conçue pour être insérée dans une capsule cristallinienne d'un œil et pour être mise en vibration pour émulsifier un cristallin de l'œil ;
un canal d'irrigation (33), conçu pour l'écoulement d'un fluide d'irrigation dans la capsule cristallinienne ; et
un canal d'aspiration (44), conçu pour retirer au moins un fluide oculaire de la capsule cristallinienne ; et **caractérisé par**
un ensemble de captage (55), qui est conçu pour être accouplé à une extrémité proximale de la sonde de phacoémulsification et est configuré pour capter une pression du fluide d'irrigation, dans lequel l'ensemble de captage comprend un ensemble de captage de pression, PSA, accouplé entre un tube d'irrigation (34) et le canal d'irrigation et configuré pour capter la pression du fluide d'irrigation s'écoulant entre le tube d'irrigation et le canal d'irrigation, dans lequel le PSA comprend : (i) un tube creux (65), qui est inséré entre le canal d'irrigation et le tube d'irrigation, et est conçu pour permettre un écoulement au moins du fluide d'irrigation, et (ii) un dispositif de captage de pression, PSD (67), qui est placé en contact direct avec le fluide d'irrigation s'écoulant dans le tube creux et est configuré pour produire un signal de pression indiquant la pression du fluide d'irrigation s'écoulant entre le tube d'irrigation et le canal d'irrigation, et dans lequel le tube creux comprend, un premier raccord luer (64) accouplé au canal d'irrigation, et un deuxième raccord luer (62) accouplé au tube d'irrigation, et dans lequel le PSD est positionné entre le premier raccord luer et le deuxième raccord luer.

5. Système de phacoémulsification selon la revendication 1 dans lequel le premier raccord est un premier raccord luer, et le deuxième raccord est un deuxième raccord luer.

6. Système de phacoémulsification selon la revendication 4, comprenant en outre un fil (68), qui est connecté au PSD et est configuré pour transmettre le signal de pression à un processeur d'une console de phacoémulsification.

7. Système de phacoémulsification selon la revendication 1, dans lequel l'ensemble de captage comprend en outre un second ensemble de captage, dans lequel le second ensemble comprend un second ensemble de captage de pression, PSA, accouplé entre le tube d'irrigation et le canal d'irrigation et configuré pour capter la pression du fluide d'irrigation s'écoulant entre le tube d'irrigation et le canal d'irrigation.

8. Système de phacoémulsification selon la revendication 7, dans lequel le second PSA comprend : (i) un second tube creux, qui est inséré entre le canal d'irrigation et le tube d'irrigation, et est conçu pour permettre un écoulement au moins du fluide d'irrigation, et (ii) un second dispositif de captage de pression, PSD, qui est placé en contact direct avec le fluide d'irrigation s'écoulant dans le second tube creux et est configuré pour produire un signal de pression indiquant la pression du fluide d'irrigation s'écoulant entre le tube d'irrigation et le canal d'irrigation.

9. Système de phacoémulsification selon la revendication 8, dans lequel le second tube creux comprend un troisième raccord accouplé au canal d'irrigation, et un quatrième raccord accouplé au tube d'irrigation, et dans lequel le second PSD est positionné entre le troisième raccord et le quatrième raccord, facultativement dans lequel le troisième raccord est un troisième raccord luer, et le quatrième raccord est un quatrième raccord luer.

10. Système de phacoémulsification (10), comprenant :
une sonde de phacoémulsification (12), comprenant :
une aiguille (16), qui est conçue pour être insérée dans une capsule cristallinienne d'un œil et pour être mise en vibration pour émulsifier un cristallin de l'œil ;
un canal d'irrigation destiné à l'écoulement de fluide d'irrigation dans la capsule cristallinienne ; et
un canal d'aspiration permettant de retirer au moins un fluide oculaire de la capsule cristallinienne ; et **caractérisé par** un ensemble raccord (80), comprenant :
un logement, comprenant : (i) un passage d'irrigation (81), permettant un raccordement entre le canal d'irrigation (33) et un tube d'irrigation (34) et conçu pour faire passer le fluide d'irrigation dans le canal d'irrigation, et (ii) un passage d'aspiration (83), permettant un raccordement entre le canal d'aspiration (44) et un tube d'aspiration (46) et conçu pour faire passer au moins le fluide oculaire à l'écart du canal d'aspiration vers le tube d'aspiration ; et
un ou plusieurs capteurs (87), contenus dans le logement et configurés pour mesurer au moins l'une parmi la pression de fluide et la température de fluide au niveau de l'un et/ou l'autre du canal d'irrigation et du canal d'aspiration.

11. Système de phacoémulsification selon la revendication 10, comprenant en outre un connecteur électrique (86), qui est configuré pour connecter et déconnecter l'ensemble raccord et la sonde de phacoémulsification, et dans lequel, lorsque l'ensemble raccord est déconnecté de la sonde de phacoémulsification, le fonctionnement de la sonde de phacoémulsification est désactivé.

12. Système de phacoémulsification selon la revendication 10, dans lequel le ou les capteurs comprennent un premier capteur de pression disposé dans le passage d'irrigation et un second capteur de pression disposé dans le passage d'aspiration.

13. Système de phacoémulsification selon la revendication 10, comprenant en outre une ou plusieurs valves (100), dans lequel la ou les valves sont accouplées au passage d'irrigation ou au passage d'aspiration et conçues pour commander un écoulement du fluide oculaire ou du fluide d'irrigation.

14. Procédé permettant de produire un ensemble raccord, le procédé comprenant :
la réception d'un logement, comprenant : (i) un passage d'irrigation, raccordé entre un canal d'irrigation d'une sonde de phacoémulsification et un tube d'irrigation permettant de faire passer un fluide d'irrigation dans le canal d'irrigation, et (ii) un passage d'aspiration, se raccordant entre un canal d'aspiration et un tube d'aspiration permettant de faire passer au moins un fluide oculaire à l'écart du canal d'aspiration vers le tube d'aspiration ; et
la mise en place, dans le logement, d'un ou plusieurs capteurs permettant de mesurer au moins l'une parmi la pression de fluide et la température de fluide au niveau de l'un et/ou l'autre du canal d'irrigation et du canal d'aspiration.

15. Procédé selon la revendication 14, comprenant en outre :
l'accouplement au logement, d'un connecteur électrique, permettant de connecter et de déconnecter l'ensemble raccord et la sonde de phacoémulsification, et dans lequel, lorsque l'ensemble raccord est déconnecté de la sonde de phacoémulsification, le fonctionnement de la sonde de phacoémulsification est désactivé, facultativement dans lequel la mise en place du ou des capteurs comprend la mise en place : (i) d'un premier capteur de pression dans le passage d'irrigation, et (ii) d'un second capteur de pression dans le passage d'aspiration.
